# FASCICULE DE BREVET EUROPEEN

(11) **EP 0 686 024 B1**
(45) Date de publication et mention de la délivrance du brevet: **07.05.1997**
(21) Numéro de dépôt: 94906950.4
(22) Date de dépôt: 16.02.1994
(51) Int. Cl.: A61K 7/06, A61K 7/48

(54) **ASSOCIATION EPAISSISSANTE A BASE DE GOMME DE GUAR OU DE CELLULOSE NON-IONIQUE ET D'UN POLYMERE RETICULE ET COMPOSITION COSMETIQUE OU DERMATOLOGIQUE POUR LE TRAITEMENT DES CHEVEUX OU DE LA PEAU CONTENANT UNE TELLE ASSOCIATION**
VERDICKUNGSMITTEL ENTHALTEND EINE KOMBINATION AUS GARGUMMI ODER NICHTIONISCHE CELLULOSE UND EINEM VERNETZTEN POLYMER UND KOSMETISCHE ODER DERMATOLOGISCHE ZUSAMMENSETZUNGEN ZUR HAAR UND HAUTBEHANDLUNG, DIE EINE SOLCHE KOMBINATION ENTHALTEN
THICKENING MIXTURE BASED ON GUAR GUM OR NON-IONIC CELLULOSE AND A CROSS-LINKED POLYMER, AND COSMETIC OR DERMATOLOGICAL HAIR- OR SKIN-CARE COMPOSITION CONTAINING SAID MIXTURE

(30) Priorité: 23.02.1993 FR 9302065
(43) Date de publication de la demande: 13.12.1995
(73) Titulaire: L'OREAL, 75008 Paris (FR)
(72) Inventeur: DUPUIS, Christine, F-75018 Paris (FR)
(74) Mandataire: Casalonga, Axel
(86) Numéro de dépôt international: FR9400170
(87) Numéro de publication internationale: WO9418935

(56) Documents cités:
- EP-A- 0 152 095
- EP-A- 0 200 620
- EP-A- 0 524 434
- WO-A-92/21316

## Description

L'invention concerne une association épaississante à base de gommes de guar ou de cellulose non-ionique, sans groupe hydrophobe et de polymères réticulés particuliers et une composition cosmétique ou dermatologique pour le traitement des cheveux ou de la peau contenant une telle association.

Afin d'apporter de la douceur aux cheveux ou à la peau ou encore de faciliter le démêlage des cheveux, on utilise en cosmétique ou en dermatologie certains polymères réticulés tels que des copolymères ou polymères d'acrylamide réticulés. Ces polymères présentent des viscosités permettant d'obtenir des propriétés épaississantes appropriées pour les formulations cosmétiques et dermatologiques, de bonnes propriétés de douceur pour les cheveux ou la peau et un toucher agréable.

Cependant, la viscosité de ces polymères réticulés est très sensible aux additifs tels que les alcools, certains polymères anioniques ou cationiques ou certains agents antipelliculaires. L'ajout de ces additifs peut provoquer des phénomènes de fluidification indésirables pour la texture des formulations cosmétiques ou dermatologiques contenant ces polymères réticulés.

La demanderesse a découvert d'une manière surprenante qu'en associant à certains polymères réticulés des épaississants particuliers choisis parmi les gommes de guar et les gommes de cellulose non-ioniques sans groupe hydrophobe, on observait un effet de synergie de viscosité des polymères réticulés permettant de surmonter les inconvénients évoqués ci-dessus.

L'association particulière conforme à la présente invention, permet de préparer des compositions cosmétiques ou dermatologiques à base de polymères réticulés sous forme de gel, de crème, d'émulsion ou de dispersion, dont les propriétés rhéologiques sont sensiblement améliorées.

Un objet de l'invention est donc constitué par une association épaississante à base de gomme de guar ou de cellulose non-ionique sans groupe hydrophobe et de polymères réticulés particuliers.

Un objet de l'invention concerne également une composition cosmétique ou dermatologique contenant une telle association.

Un autre objet concerne des procédés de traitement cosmétique des cheveux ou de la peau, mettant en oeuvre ces compositions selon l'application désirée.

D'autres objets apparaîtront à la lumière de la description et des exemples qui suivent.

La présente invention concerne principalement une association épaississante, caractérisée par le fait qu'elle comprend dans un milieu aqueux :
a) un composant (A) constitué d'au moins une gomme de guar ou de cellulose non-ionique, sans groupe hydrophobe, possédant une viscosité en solution à 1,5% en poids dans l'eau, mesurée au DRAGE module 2 à 25°C supérieure à 15 cps;
b) un composant (B) constitué d'au moins un polymère réticulé choisi parmi :
   (i) les copolymères d'acrylamide et d'acrylate d'ammonium;
   (ii) les copolymères d'acrylamide et d'acide-2-acrylamido 2-méthylpropane sulfonique partiellement ou totalement neutralisé;
   (iii) les copolymères d'acrylamide et de chlorure de méthacryloyl oxyéthyl triméthylammonium;
   (iv) les homopolymères de chlorure de méthacryloyloxyéthyl-triméthylammonium.

Le rapport en poids en matière active polymère réticulé/gomme de guar ou cellulose non-ionique, est compris entre 0,2 et 10, et de préférence entre 1 et 5.

Parmi les gommes de guar utilisées selon la présente invention, on peut citer :
- la gomme de guar hydroxypropylée vendue sous la dénomination "JAGUAR HP8" par la Société MEY HALL;
- la gomme de guar vendue sous la dénomination "GUARGEL D/15" par la SOCIETE FRANCAISE DES COLLOIDES.

Parmi les gommes de cellulose non-ionique utilisées conformément à la présente invention, on peut mentionner :
- la méthylhydroxypropylcellulose vendue sous la dénomination "METHOCEL F₄M STANDARD" par la Société DOW CHEMICAL;
- la méthylcellulose vendue sous la dénomination "METHYL CELLULOSE 200" par la Société LASERSON SABETAY;
- l'hydroxyéthylcellulose vendue sous la dénomination "NATROSOL HHR" par la Société AQUALON;
- l'hydroxypropylcellulose vendue sous la dénomination "KLUCEL H" par la Société AQUALON;
- la méthylhydroxyéthylcellulose vendue sous la dénomination "TYLOSE MH 300" par la Société HOECHST.

Le copolymère réticulé d'acrylamide/acrylate d'ammonium, utilisé conformément à la présente invention, est plus particulièrement un copolymère acrylamide/acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcools de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce copolymère réticulé sous forme d'émulsion eau-dans-huile, constituée de 30% en poids dudit copolymère, 25% en poids d'huile de paraffine, 4% en poids de mélange de stéarate de sorbitan et d'un dérivé éthoxylé hydrophile, et 41% en poids d'eau. Une telle émulsion est commercialisée sous le nom "PAS 5161" ou encore "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55% en moles d'acrylamide et de 30 à 45% en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions huile-dans-eau contenant de 35 à 40% en poids de ce copolymère, de 15 à 25% en poids d'un mélange d'hydrocarbures isoparaffiniques en C₁₂-C₁₃, de 3 à 8% en poids de lauryléther de polyéthylèneglycol à 7 moles d'oxyde d'éthylène et d'eau. Une telle émulsion est commercialisée sous le nom de "SEPIGEL 305" par la Société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (20/80 en poids) sous forme de dispersion contenant 50% en poids dudit copolymère dans de l'huile minérale. Cette dispersion est commercialisée sous le nom de "SALCARE SC92" par la Société ALLIED COLLOIDS.

L'homopolymère de chlorure de méthacryloyl oxyéthyl triméthylammonium est réticulé par un composé à insaturation oléfinique, tel que ceux définis précédemment, en particulier le méthylène-bis acrylamide. On utilise plus particulièrement l'homopolymère sous forme de dispersion contenant 50% en poids dudit homopolymère dans de l'huile minérale. Cette dispersion est vendue sous la dénomination "SALCARE SC95" par la Société ALLIED COLLOIDS.

Un autre objet de l'invention concerne des compositions cosmétiques ou dermatologiques contenant pour le traitement des cheveux ou de la peau contenant dans un milieu aqueux physiologiquement acceptable, au moins l'association des composants (A) et (B) telle que définie ci-dessus.

Les gommes de guar ou de cellulose non-ionique conformes à l'invention sont présentes dans ces compositions à des concentrations en matière active comprises entre 0,1 et 10% en poids et de préférence entre 0,2 et 5% en poids par rapport au poids total de la composition.

Les polymères réticulés de l'invention sont présents dans les compositions dans des concentrations en matière active comprises entre 0,1 et 10% en poids par rapport au poids total de la composition, de préférence entre 0,5 et 7%.

Les compositions cosmétiques ou dermatologiques, conformes à l'invention, se présentent sous forme de gel, d'émulsion ou de dispersion vésiculaire.

Lorsque la composition se présente sous forme de gel, le milieu physiologiquement acceptable est constitué par de l'eau ou un mélange d'eau et d'alcool inférieur, en particulier l'éthanol.

Lorsque la composition se présente sous forme d'émulsion, les constituants de l'association conforme à l'invention sont présents dans la phase aqueuse. L'émulsion est préparée à partir de tensio-actifs et d'huiles bien connus dans l'art antérieur.

Les compositions conformes à l'invention peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans l'article BANGHAM, STANDISH & WATKINS, J. Mol. Biol., 13, 238 (1965) ou dans le brevet FR-2.315.991 et 2.416.008 de la demanderesse.

Les différents types de procédés de préparation sont décrits dans "Les liposomes en biologie cellulaire et pharmacologie", Edition INSERM/John Libbery Eurotext, 1987, pages 6 à 18.

Les constituants de l'association conforme à l'invention sont dans la phase aqueuse de la dispersion.

Les compositions selon l'invention peuvent contenir en plus des adjuvants habituellement utilisés en cosmétique ou dermatologie, tels que des parfums, des colorants, des conservateurs, des agents séquestrants, des huiles végétales, animales ou synthétiques, des filtres solaires, des anti-radicaux libres, des agents tensio-actifs, des polymères naturels ou synthétiques, anioniques, non-ioniques, cationiques ou amphotères, des protéines quaternisées ou non, des silicones, des agents de conditionnement, des agents anti-gras, des agents hydratants, des propulseurs.

Les compositions cosmétiques ou dermatologiques destinées au traitement et au soin des cheveux, peuvent être utilisées sous forme de gel ou crème capillaire anti-chute ou antipelliculaire, de gel de coiffage.

Les compositions selon l'invention destinées au traitement et au soin de la peau, peuvent être conditionnées sous forme de gel ou de crème pour le soin de la peau; de produit pour le rasage; de crème ou de gel solaire.

Les compositions selon l'invention peuvent être appliquées par voie topique en dermatologie. Elles contiennent en une quantité efficace une substance active sur le plan dermatologique telle que la vitamine A, les caroténoïdes, les pigments naturels, les rétinoïdes, les dépigmentants, les agents anti-séborrhéiques, anti-acnéiques, anti-inflammatoires, anti-pelliculaires ou antichutes.

Un procédé de traitement cosmétique des cheveux, selon l'invention, consiste à appliquer les compositions telles que définies ci-dessus sur les cheveux, suivant l'usage envisagé puis à rincer éventuellement.

Un procédé de traitement cosmétique de la peau, selon l'invention, consiste à appliquer sur celle-ci une composition telle que définie précédemment et à rincer éventuellement.

Les exemples qui suivent sont destinés à illustrer l'invention sans pour autant présenter un caractère limitatif.

### EXEMPLES DE PREPARATION

### EXEMPLE 1

On prépare un gel de coiffage de composition suivante :

| | |
|---|---|
| - Emulsion de copolymère réticulé acrylamide/2-acrylamido 2-méthyl propane sulfonate de sodium, vendue à environ 40% en copolymère sous la dénomination "SEPIGEL 305" par la Société SEPPIC | 1 g en copolymère |
| - Hydroxypropylcellulose vendue sous la dénomination "KLUCEL H" par la Société AQUALON | 1 g |
| - Ethanol | 8,5 g |
| - Parfum, colorant, conservateur qs | |
| - Eau déminéralisée qsp | 100 g |

### EXEMPLE 2

On prépare un gel de coiffage de composition suivante :

| | |
|---|---|
| - Emulsion eau-dans-huile de copolymère réticulé acrylamide/acrylate d'ammonium, vendue à 30% en copolymère sous la dénomination "PAS 5161" par la Société HOECHST | 3 g en copolymère |
| - Copolymère vinylméthyléther/anhydride maléique monoestérifié avec le butanol, vendu à 50% de matière active (MA) dans l'éthanol sous la dénomination "GANTREZ ES 425" par la Société ISP (neutralisé à 100% par le 2-amino 2-méthyl 1-propanol) | 1 g MA |
| - Hydroxypropylméthylcellulose vendue sous la dénomination "METHOCEL F 4M STANDARD" par la Société DOW CHEMICAL | 2 g |
| - Parfum, colorant, conservateur qs | |
| - Eau déminéralisée qsp | 100 g |

### EXEMPLE 3

On prépare un gel de coiffage de composition suivante :

| | |
|---|---|
| - Dispersion dans l'huile minérale de copolymère réticulé acrylamide/chlorure de méthacryloyloxyéthyl triméthyl ammonium, vendu à 50% en copolymère sous la dénomination "SALCARE SC 92" par la Société ALLIED COLLOIDS | 2,5 g en copolymère |
| - Gomme de guar hydroxypropylée, vendue sous la dénomination "JAGUAR HP 8" par la Société MEYHALL | 1,5 g |
| - Copolymère vinylpyrrolidone/chlorure de méthacrylamidopropyl triméthyl ammonium (85/15), vendu en solution aqueuse à 20% de matière active sous la dénomination "GAFQUAT HS 100" par la Société ISP | 0,5 g MA |
| - Hydrolysat de protéines de blé, vendu en solution aqueuse à 20% de matière active sous la dénomination "HYDROTRITICUM 2000" par la Société CRODA | 0,2 g MA |
| - Polydiméthylsiloxane oxyéthyléné, vendu sous la dénomination "SILWET L 7602" par la Société UNION CARBIDE | 0,2 g |
| - Parfum, colorant, conservateur qs | |
| - Eau déminéralisée qsp | 100 g |

### EXEMPLE 4

On prépare un gel antichute de composition suivante :

| | |
|---|---|
| - Emulsion de copolymère réticulé acrylamide/2-acrylamido 2-méthyl propane sulfonate de sodium, vendue à environ 40% en copolymère sous la dénomination "SEPIGEL 305" par la Société SEPPIC | 3 g en copolymère |
| - Méthylcellulose vendue sous la dénomination "METHYL CELLULOSE 200" par la Société LASERSON SABETAY | 1 g |
| - Nicotinate de méthyle | 0,1 g |
| - Parfum, colorant, conservateur qs | |
| - Eau déminéralisée qsp | 100 g |

### EXEMPLE 5

On prépare un gel antipelliculaire de composition suivante :

| | |
|---|---|
| - Emulsion de copolymère réticulé acrylamide/2-acrylamido 2-méthyl propane sulfonate de sodium, vendue à environ 40% en copolymère sous la dénomination "SEPIGEL 305" par la Société SEPPIC | 10 g en copolymère |
| - Hydroxypropylcellulose vendue sous la dénomination "KLUCEL H" par la Société AQUALON | 1 g |
| - 1-hydroxy 4-méthyl 6-(2,4,4-triméthylpentyl)2-(1H)pyridinone, sel de monoéthanolamine, vendu sous la dénomination "OCTOPIROX" par la Société HOECHST | 0,1 g |
| - Ethanol | 35,5 g |
| - Parfum, colorant, conservateur qs | |
| - Eau déminéralisée qsp | 100 g |

### EXEMPLE 6

On prépare un gel de coiffage de composition suivante :

| | |
|---|---|
| - Dispersion dans l'huile minérale de copolymère réticulé acrylamide/chlorure de méthacryloyloxyéthyl triméthyl ammonium, vendu à 50% en copolymère sous la dénomination "SALCARE SC 92" par la Société ALLIED COLLOIDS | 1 g en copolymère |
| - Hydoxypolyméthylcellulose vendue sous la dénomination "METHOCEL F4M STANDARD" par la Société DOW CHEMICAL | 0,3 g |
| - Pyrrolidone carboxylate de chitosane, vendu sous la dénomination "KYTAMER PC" par la Société AMERCHOL | 0,25 g |
| - Parfum, colorant, conservateur qs | |
| - Eau qsp | 100 g |

### EXEMPLE 7

On prépare un gel de coiffage de composition suivante :

| | |
|---|---|
| - Emulsion de copolymère réticulé acrylamide/2-acrylamido 2-méthyl propane sulfonate de sodium, vendue à environ 40% en copolymère sous la dénomination "SEPIGEL 305" par la Société SEPPIC | 1 g en copolymère |
| - Hydroxypropylcellulose vendue sous la dénomination "KLUCEL H" par la Société AQUALON | 1 g |
| - Parfum, colorant, conservateur qs | |
| - Eau déminéralisée qsp | 100 g |

### EXEMPLE 8

On prépare un gel de coiffage de composition suivante :

| | |
|---|---|
| - Emulsion de copolymère réticulé acrylamide/2-acrylamido 2-méthyl propane sulfonate de sodium, vendue à environ 40% en copolymère sous la dénomination "SEPIGEL 305" par la Société SEPPIC | 1 g en copolymère |
| - Gomme de guar non ionique, vendue sous la dénomination "GUARGEL D/15" par la SOCIETE FRANCAISE DES COLLOIDES | 1 g |
| - Parfum, colorant, conservateur qs | |
| - Eau déminéralisée qsp | 100 g |

### EXEMPLE 9

On prépare un gel de coiffage de composition suivante :

| | |
|---|---|
| - Emulsion eau-dans-huile de copolymère réticulé acrylamide/acrylate d'ammonium, vendue à 30% en copolymère sous la dénomination "PAS 5161" par la Société HOECHST | 1 g en copolymère |
| - Hydroxypropylcellulose vendue sous la dénomination "KLUCEL H" par la Société AQUALON | 1 g |
| - Parfum, colorant, conservateur qs | |
| - Eau déminéralisée qsp | 100 g |

### EXEMPLE 10

On prépare un gel de coiffage de composition suivante :

| | |
|---|---|
| - Dispersion dans l'huile minérale de copolymère réticulé acrylamide/chlorure de méthacryloyloxyéthyl triméthyl ammonium, vendu à 50% en copolymère sous la dénomination "SALCARE SC 92" par la Société ALLIED COLLOIDS | 1 g en copolymère |
| - Hydroxypropylcellulose vendue sous la dénomination "KLUCEL H" par la Société AQUALON | 1 g |
| - Parfum, colorant, conservateur qs | |
| - Eau déminéralisée qsp | 100 g |

### EXEMPLE 11

On prépare un gel de coiffage de composition suivante :

| | |
|---|---|
| - Emulsion eau-dans-huile de copolymère réticulé acrylamide/acrylate d'ammonium, vendue à 30% en copolymère sous la dénomination "PAS 5161" par la Société HOECHST | 1 g en copolymère |
| - Gomme de guar non ionique, vendue sous la dénomination "GUARGEL D/15" par la SOCIETE FRANCAISE DES COLLOIDES | 1 g |
| - Parfum, colorant, conservateur qs | |
| - Eau déminéralisée qsp | 100 g |

### EXEMPLE 12

On prépare un gel de coiffage de composition suivante :

| | |
|---|---|
| - Dispersion dans l'huile minérale de copolymère réticulé acrylamide/chlorure de méthacryloyloxyéthyl triméthyl ammonium, vendu à 50% en copolymère sous la dénomination "SALCARE SC 92" par la Société ALLIED COLLOIDS | 1 g en copolymère |
| - Gomme de guar non ionique vendue sous la dénomination GUARGEL D/15 par la SOCIETE FRANCAISE DES COLLOIDES | 1 g |
| - Parfum, colorant, conservateur qs | |
| - Eau déminéralisée qsp | 100 g |

### EXEMPLE 13

On prépare un gel de coiffage de composition suivante :

| | |
|---|---|
| - Dispersion dans l'huile minérale d'homopolymère réticulé de chlorure de méthacryloyloxyéthyl triméthyl ammonium, vendu à 50% en polymère sous la dénomination "SALCARE SC 95" par la Société ALLIED COLLOIDS | 2 g en polymère |
| - Hydroxypropylméthylcellulose vendue sous la dénomination "METHOCEL F4M STANDARD" par la Société DOW CHEMICAL | 1 g |
| - Parfum, colorant, conservateur qs | |
| - Eau qsp | 100 g |

### EXEMPLE 14

On prépare un gel de soin pour la peau de composition suivante :

| | |
|---|---|
| - Dispersion dans l'huile minérale de copolymère réticulé acrylamide/chlorure de méthacryloyloxyéthyl triméthyl ammonium, vendu à 50% en copolymère sous la dénomination "SALCARE SC 92" par la Société ALLIED COLLOIDS | 0,5 g en copolymère |
| - Hydroxypropylcellulose vendue sous la dénomination "KLUCEL H" par la Société AQUALON | 0,25 g |
| - Huile de germe de maïs | 10 g |
| - Eau qsp | 100 g |

### EXEMPLE 15

On prépare une crème de protection solaire de composition suivante :

| | |
|---|---|
| - Dispersion dans l'huile minérale de copolymère réticulé acrylamide/chlorure de méthacryloyloxyéthyl triméthyl ammonium, vendu à 50% en copolymère sous la dénomination "SALCARE SC 95" par la Société ALLIED COLLOIDS | 1 g en copolymère |
| - Hydroxypropylméthylcellulose vendue sous la dénomination "METHOCEL F4M STANDARD" par la Société DOW CHEMICAL | 0,25 g |
| - Huile de colza | 10 g |
| - 4-(tert.butyl)4'-méthoxy dibenzoylméthane | 5 g |
| - Eau qsp | 100 g |

## Revendications

1. Association épaississante, caractérisée par le fait qu'elle comprend dans un milieu aqueux :
a) un composant (A) constitué d'au moins une gomme de guar ou de cellulose non-ionique, sans groupe hydrophobe, possédant une viscosité en solution à 1,5% en poids dans l'eau, mesurée au DRAGE module 2 à 25°C supérieure à 15.10⁻³ Pa.s;
b) un composant (B) constitué d'au moins un polymère réticulé choisi parmi :
(i) les copolymères d'acrylamide et d'acrylate d'ammonium;
(ii) les copolymères d'acrylamide et d'acide-2-acrylamido 2-méthylpropane sulfonique partiellement ou totalement neutralisé;
(iii) les copolymères d'acrylamide et de chlorure de méthacryloyl oxyéthyl triméthylammonium;
(iv) les homopolymères de chlorure de méthacryloyloxyéthyl-triméthylammonium;
le rapport en poids en matière active polymère réticulé/gomme de guar ou de cellulose étant compris entre 0,2 et 10.

2. Association selon la revendication 1, caractérisée par le fait que la gomme de guar ou de cellulose du composant (A) est choisie parmi les gommes de guar hydroxypropylées, la gomme de guar, la méthylhydroxypropylcellulose, la méthylcellulose, l'hydroxypropylcellulose, l'hydroxyéthylcellulose, la méthylhydroxyéthylcellulose dont les viscosités en solution à 1,5% en poids dans l'eau mesurées au DRAGE module 2 à 25°C sont supérieures à 15.10⁻³ Pa.s.

3. Association selon la revendication 1 ou 2, caractérisée par le fait que le polymère du composant (B) est réticulé par un composé à polyinsaturation oléfinique choisi parmi le divinylbenzène, le tétraallyloxyéthane, le méthylène bis-acrylamide, l'éther diallylique, les éthers polyallylpolyglycéryliques ou les éthers allyliques d'alcools de la série des sucres.

4. Association selon l'une quelconque des revendications 1 à 3, caractérisée par le fait que le composant (B) est un copolymère réticulé d'acrylamide/acrylate d'ammonium (5/95 en poids) sous forme d'émulsion eau-dans-huile, comprenant 30% en poids dudit copolymère, 25% en poids d'huile de paraffine, 4% en poids de mélange de stéarate de sorbitan et d'un dérivé éthoxylé hydrophile et 41% en poids d'eau.

5. Association selon l'une quelconque des revendications 1 à 3, caractérisée par le fait que le composant (B) est un copolymère réticulé d'acrylamide/acide 2-acrylamido 2-méthylpropane sulfonique partiellement ou totalement neutralisé par la soude, la potasse, l'ammoniaque ou une amine, sous forme d'émulsion huile-dans-eau contenant 35 à 40% en poids dudit copolymère, 15 à 25% en poids d'un mélange d'hydrocarbures isoparaffiniques en C₁₂-C₁₃, de 3 à 8% en poids de lauryléther de polyéthylèneglycol à 7 moles d'oxyde d'éthylène et d'eau.

6. Association selon l'une quelconque des revendications 1 à 3, caractérisée par le fait que le composant (B) est un copolymère réticulé d'acrylamide/chlorure de méthacryloyl oxyéthyl triméthyl ammonium (20/80 en poids) sous forme de dispersion contenant 50% dudit copolymère dans de l'huile minérale.

7. Association selon l'une quelconque des revendications 1 à 3, caractérisée par le fait que le composant (B) est un homopolymère de chlorure de méthacryloyloxyéthyl triméthylammonium réticulé par le méthylène-bis-acrylamide sous forme de dispersion contenant 50% dudit homopolymère dans de l'huile minérale.

8. Composition cosmétique ou dermatoloqique pour le traitement des cheveux ou de la peau, caractérisée par le fait qu'elle contient dans un milieu aqueux physiologiquement acceptable, au moins l'association des composants (A) et (B) telle que définie dans l'une quelconque des revendications 1 à 7.

9. Composition selon la revendication 8, caractérisée par le fait que la gomme de guar ou de cellulose non-ionique du composant (A) est présente dans des concentrations en matière active comprises entre 0,1 et 10% en poids, de préférence entre 0,2 et 5% en poids et que le polymère réticulé du composant (B) est présent dans des proportions comprises entre 0,1 et 10% en poids, de préférence entre 0,5 et 7% en poids, les pourcentages en poids étant définis par rapport au poids total de la composition.

10. Composition selon la revendication 8 ou 9, caractérisée par le fait qu'elle se présente sous forme de gel, d'émulsion ou de dispersion vésiculaire de lipides amphiphiles ioniques ou non-ioniques.

11. Composition selon l'une quelconque des revendications 8 à 10, caractérisée par le fait que le milieu physiologiquement acceptable est constitué par de l'eau ou est un milieu hydroalcoolique.

12. Composition selon l'une quelconque des revendications 8 à 11, caractérisée par le fait qu'elle contient en plus un additif habituellement utilisé en cosmétique ou dermatologie, choisi parmi les parfums, les colorants, les conservateurs, les agents séquestrants, les huiles végétales, animales ou synthétiques, des filtres solaires, des anti-radicaux libres, des agents tensio-actifs, des polymères naturels ou synthétiques, anioniques, non-ioniques, amphotères ou cationiques, des protéines quaternisées ou non, des agents de conditionnement, des propulseurs, des silicones, des agents hydratants, la vitamine A, les caroténoïdes, les pigments naturels, les rétinoïdes, les dépigmentants, les agents antiséborrhéïques, anti-acnéiques, anti-inflammatoires, anti-pelliculaires ou antichutes.

13. Composition selon l'une quelconque des revendications 8 à 12, destinée au traitement des cheveux, caractérisée par le fait qu'elle est conditionnée sous forme de gel ou de crème capillaire anti-chute ou antipelliculaire ou de gel de coiffage.

14. Composition selon l'une quelconque des revendications 8 à 13, destinée au traitement de la peau, caractérisée par le fait qu'elle est conditionnée sous forme de gel ou de crème pour le soin; de produit de rasage; de crème ou de gel solaire.

15. Procédé de traitement cosmétique des cheveux, caractérisé par le fait qu'on applique une composition telle que définie dans la revendication 13, sur les cheveux et que l'on rince éventuellement.

16. Procédé de traitement cosmétique de la peau, caractérisé par le fait qu'on applique sur la peau une composition telle que définie dans la revendication 14.

## Claims

1. A thickening combination, wherein it comprises, in an aqueous medium:
a) a component (A) comprising at least one guar gum or nonionic cellulose gum, having no hydrophobic group, having a viscosity, as a 1.5 weight % solution in water, measured with a Drage unit 2 at 25°C, greater than 15 × 10⁻³ Pa·s;
b) a component (B) comprising at least one crosslinked polymer chosen from:
(i) acrylamide and ammonium acrylate copolymers;
(ii) acrylamide and partially or totally neutralized 2-acrylamido-2-methylpropanesulfonic-acid [sic] copolymers;
(iii) acrylamide and methacryloyloxyethyltrimethylammonium chloride copolymers;
(iv) methacryloyloxyethyltrimethylammonium chloride homopolymers.
the crosslinked polymer/guar gum or cellulose gum ratio, as weight of active material, being between 0.2 and 10.

2. The combination as claimed in claim 1, wherein the guar gum or cellulose gum of the component (A) is chosen from hydroxypropylated guar gums, guar gum, methylhydroxypropylcellulose, methylcellulose, hydroxypropylcellulose, hydroxyethylcellulose or methylhydroxyethylcellulose, the viscosities of which, as a 1.5 weight % solution in water, measured with a Drage unit 2 at 25°C, are greater than 15 × 10⁻³ Pa·s.

3. The combination as claimed in claim 1 or 2, wherein the polymer of the component (B) is crosslinked by a compound possessing olefinic polyunsaturation chosen from divinylbenzene, tetraallyloxyethane, methylenebisacrylamide, diallyl ether, polyallyl polyglyceryl ethers or allyl ethers of alcohols from the sugar series.

4. The combination as claimed in any one of claims 1 to 3, wherein the component (B) is a crosslinked acrylamide-ammonium acrylate (5/95 by weight) copolymer in the form of a water-in-oil emulsion, comprising 30 weight % of said copolymer, 25 weight % of liquid paraffin, 4 weight % of a mixture of sorbitan stearate and of a hydrophilic ethoxylated derivative and 41 weight % of water.

5. The combination as claimed in any one of claims 1 to 3, wherein the component (B) is a crosslinked acrylamide-2-acrylamido-2-methylpropanesulfonic acid copolymer partially or totally neutralized by sodium hydroxide, potassium hydroxide, ammonia or an amine, in the form of an oil-in-water emulsion containing 35 to 40 weight % of said copolymer, 15 to 25 weight % of a mixture of C₁₂-C₁₃ isoparaffin hydrocarbons, from 3 to 8 weight % of lauryl ether of poly(ethylene glycol) containing 7 mol of ethylene oxide, and water.

6. The combination as claimed in any one of claims 1 to 3, wherein the component (B) is a crosslinked acrylamide-methacryloyloxyethyltrimethylammonium chloride (20/80 by weight) copolymer in the form of a dispersion containing 50 % of said copolymer in mineral oil.

7. The combination as claimed in any one of claims 1 to 3, wherein the component (B) is a methacryloyloxyethyltrimethylammonium chloride homopolymer crosslinked by methylenebisacrylamide in the form of a dispersion containing 50 % of said homopolymer in mineral oil.

8. A cosmetic or dermatological hair or skin treatment composition, wherein it contains, in a physiologically acceptable aqueous medium, at least the combination of the components (A) and (B) as defined in any one of claims 1 to 7.

9. The composition as claimed in claim 8, wherein the guar gum or nonionic cellulose gum of the component (A) is present in active material concentrations of between 0.1 and 10 weight %, preferably between 0.2 and 5 weight %, and wherein the crosslinked polymer of the component (B) is present in proportions of between 0.1 and 10 weight %, preferably between 0.5 and 7 weight %, the percentages by weight being defined with respect to the total weight of the composition.

10. The composition as claimed in claim 8 or 9, wherein it is provided in the gel or emulsion form or in the form of a vesicular dispersion of ionic or nonionic amphiphilic lipids.

11. The composition as claimed in any one of claims 8 to 10, wherein the physiologically acceptable medium is composed of water or is an aqueous/alcoholic medium.

12. The composition as claimed in any one of claims 8 to 11, wherein it additionally contains an additive commonly used in cosmetics or dermatology, chosen from fragrances, dyes, preservatives, sequestering agents, vegetable, animal or synthetic oils, sunscreens, agents for combating free radicals, surface-active agents, anionic, nonionic, amphoteric or cationic natural or synthetic polymers [sic], proteins, which may or may not be quaternized, conditioning agents, propellants, silicones, moisturizing agents, vitamin A, carotenoids, natural pigments, retinoids, depigmenting agents, anti-seborrheic, antiacne, anti-inflammatory or antidandruff agents or agents for combating hair loss.

13. The composition as claimed in any one of claims 8 to 12 intended for hair treatment, wherein it is packaged in the form of a hair gel or cream for combating hair loss or dandruff or of a styling gel.

14. The composition as claimed in any one of claims 8 to 13 intended for skin treatment, wherein it is packaged in the form of a gel or cream for care [sic], of a shaving product or of a sun cream or gel.

15. A process for the cosmetic treatment of hair, wherein a composition as defined in claim 13 is applied to hair and wherein rinsing is optionally carried out.

16. A process for the cosmetic treatment of the skin, wherein a composition as defined in claim 14 is applied to the skin.

## Patentansprüche

1. Verdickende Mischung,
dadurch **gekennzeichnet**, daß
sie in einem wässrigen Medium enthält:
a) einen Bestandteil (A) aus mindestens einem Guar-Gummi oder einer nicht-ionischen Cellulose, ohne hydrophobe Gruppe, welcher eine Viskosität in wässriger Lösung von 1,5 Gew.%, gemessen gemäß DRAGE-Modul 2 bei 25°C, von mehr als 15x10⁻³ Pa x s aufweist;
b) einen Bestandteil (B) aus mindestens einem Polymer, ausgewählt aus:
(i) Copolymeren aus Acrylamid und Ammoniumacrylat;
(ii) Copolymeren aus Acrylamid und teilweise oder ganz neutralisierter 2-Acrylamido-2-methylpropansulfonsäure;
(iii) Copolymeren aus Acrylamid und Methacryloyloxytrimethylammoniumchlorid;
(iv) Homopolymeren aus Methacryloyloxyethyltrimethylammoniumchlorid
wobei das Gewichtsverhältnis an Aktivmasse von vernetztem Polymer/Guar-Gummi oder Cellulose 0,2 bis 10 beträgt.

2. Mischung gemäß Anspruch 1,
dadurch **gekennzeichnet**, daß
das gummiartige Produkt aus Guar oder Cellulose des Bestandteils (A) aus hydroxypropylierten Guar-Gummiprodukten, Guar-Gummi, Methylhydroxypropylcellulose, Methylcellulose, Hydroxypropylcellulose, Hydroxyethylcellulose und Methylhydroxyethylcellulose ausgewählt ist, deren Viskositätswerte in einer wässrigen Lösung von 1,5 Gew.%, gemessen gemäß DRAGE-Modul 2 bei 25°C, mehr als 15 x 10⁻³ Pa x s beträgt.

3. Mischung gemäß Anspruch 1 oder 2,
dadurch **gekennzeichnet**, daß
das Polymer des Bestandteils (B) mit einer olefinisch mehrfach ungesättigten Verbindung vernetzt ist, die aus Divinylbenzol, Tetraallyloxyethan, Methylenbisacrylamid, Diallylether, Polyallylpolyglycerylethern oder aus Allylethern von Zuckeralkoholen ausgewählt ist.

4. Mischung gemäß einem der Ansprüche 1 bis 3,
dadurch **gekennzeichnet**, daß
der Bestandteil (B) ein vernetztes Acrylamid/Ammoniumacrylat-Copolymer (5/95, gewichtsbezogen) in Form einer Wasser-in-Öl-Emulsion ist, die 30 Gew.% des genannten Copolymers, 25 Gew.% Paraffinöl, 4 Gew.% einer Mischung aus Sorbitanstearat und einem ethoxylierten hydrophilen Derivat sowie 41 Gew.% Wasser enthält.

5. Mischung gemäß einem der Ansprüche 1 bis 3,
dadurch **gekennzeichnet**, daß
der Bestandteil (B) ein vernetztes Acrylamid/2-Acrylamido-2-methylpropansulfonsäure-Copolymer, das mit Soda, Pottasche, Ammoniak oder einem Amin teilweise oder ganz neutralisiert ist, in Form einer Öl-in-Wasser-Emulsion ist, die 35 bis 40 Gew.% des genannten Copolymer, 15 bis 25 Gew.% einer Mischung aus isoparaffinischen C₁₂₋₁₃-Kohlenwasserstoffen, 3 bis 8 Gew.% Polyethylenglycollaurylether mit 7 Mol Ethylenoxid und Wasser enthält.

6. Mischung gemäß einem der Ansprüche 1 bis 3,
dadurch **gekennzeichnet**, daß
der Bestandteil (B) ein vernetztes Acrylamid/Methacryloyloxyethyltrimethylammoniumchlorid-Copolymer (20/80, gewichtsbezogen) in Form einer Dispersion ist, die 50% des genannten Copolymer in Mineralöl enthält.

7. Mischung gemäß einem der Ansprüche 1 bis 3,
dadurch **gekennzeichnet**, daß
der Bestandteil (B) ein mit Methylenbisacrylamid vernetztes Methacryloyloxytrimethylammoniumchlorid-Homopolymer in Form einer Dispersion ist, die 50% des genannten Homopolymer in Mineralöl enthält.

8. Kosmetische oder deramtologische Zusammensetzung zur Behandlung der Haare oder der Haut,
dadurch **gekennzeichnet**, daß
sie in einem wässrigen physiologisch geeigneten Medium mindestens die in jedem der Ansprüche 1 bis 7 definierte Mischung der Bestandteile (A) und (B) enthält.

9. Zusammensetzung gemäß Anspruch 8,
dadurch **gekennzeichnet**, daß
das Gummiprodukt aus Guar oder nicht-ionischer Cellulose des Bestandteils (A) in Konzentrationen an Aktivmasse von 0,1 bis 10 und vorzugsweise von 0,2 bis 5 Gew.% und das vernetzte Polymer des Bestandteils (B) in Mengenanteilen von 0,1 bis 10 und vorzugsweise von 0,5 bis 7 Gew.% vorhanden sind, wobei die Gew.-Prozentangaben unter Bezug auf das Gesamtgewicht der Zusammensetzung definiert sind.

10. Zusammensetzung gemäß Anspruch 8 oder 9,
dadurch **gekennzeichnet**, daß
sie in Form eines Gels, einer Emulsion oder einer bläschenartigen Dispersion aus amphiphilen ionischen oder nicht-ionischen Lipiden vorliegt.

11. Zusammensetzung gemäß einem der Ansprüche 8 bis 10,
dadurch **gekennzeichnet**, daß
das physilogisch geeignete Medium aus Wasser zusammengesetzt oder ein hydroalkoholisches Medium ist.

12. Zusammensetzung gemäß einem der Ansprüche 8 bis 11,
dadurch **gekennzeichnet**, daß
sie zusätzlich ein gewöhnlich in der Kosmetik oder Dermatologie eingesetztes Additiv enthält, ausgewählt aus Parfüm-Produkten, Farbstoffen, Konservierungsstoffen, Sequestriermitteln, pflanzlichen, tierischen oder synthetischen Ölen, Sonnenfilterstoffen, Anfangmitteln für freie Radikale, oberflächenaktiven Mitteln, natürlichen oder synthetischen, anionischen, nicht-ionischen, amphoteren oder kationischen Polymeren, gegebenenfalls quaternierten Proteinen, Konditioniermitteln, Treibmitteln, Siliconen, Hydratisiermitteln, Vitamin A, Karotinoiden, natürlichen Pigmenten, Retinoiden, Entpigmentiermitteln, antiseborrheischen Mitteln, Mitteln gegen Akne, entzündungshemmenden Mitteln sowie aus Mitteln gegen Schuppen oder Haarausfall.

13. Zusammensetzung gemäß einem der Ansprüche 8 bis 12 zur Behandlung der Haare,
dadurch **gekennzeichnet**, daß
sie in Form eines für Haare vorgesehenen Gels oder einer entsprechenden Creme gegen Haarausfall oder Schuppen oder eines Frisurgels zubereitet ist.

14. Zusammensetzung gemäß einem der Ansprüche 8 bis 12 zur Behandlung der Haut,
dadurch **gekennzeichnet**, daß
sie in Form eines zur Pflege vorgesehenen Gels oder einer entsprechenden Creme, eines Produkts zur Rasur, einer Sonnencreme oder eines entsprechenden Gels zubereitet ist.

15. Verfahren zur kosmetischen Behandlung der Haare,
dadurch **gekennzeichnet**, daß
man eine in Anspruch 13 definierte Zusammensetzung auf die Haare aufbringt und gegebenenfalls spült.

16. Verfahren zur kosmetischen Behandlung der Haut,
dadurch **gekennzeichnet**, daß
man auf die Haut eine nach Anspruch 14 definierte Zusammensetzung aufträgt.
